# EUROPÄISCHE PATENTSCHRIFT

(11) **EP 3 912 706 B1**
(45) Veröffentlichungstag und Bekanntmachung des Hinweises auf die Patenterteilung: **08.03.2023**
(21) Anmeldenummer: 20181069.4
(22) Anmeldetag: 19.06.2020
(51) Int. Cl.: B01D 61/02, B01D 61/10, B01D 61/12, B01D 61/14, B01D 61/24, B01D 61/58, B01D 63/10, C02F 1/44

(54) **UMKEHROSMOSE-ANLAGE UND VERFAHREN ZUR GEWINNUNG VON REINSTWASSER**
REVERSE OSMOSIS PLANT AND METHOD FOR OBTAINING HIGH-PURITY WATER
INSTALLATION D'OSMOSE INVERSE ET PROCÉDÉ DE PRODUCTION D'EAU TRÈS PURE

(30) Priorität: 22.05.2020 DE 202020102937 U
(43) Veröffentlichungstag der Anmeldung: 24.11.2021
(73) Patentinhaber: Fidica GmbH & Co. KG, 63877 Sailauf (DE)
(72) Erfinder: Reinhart, Thomas, 63843 Niedernberg (DE); Völker, Manfred, 63825 Blankenbach (DE)
(74) Vertreter: Flosdorff, Jürgen

(56) Entgegenhaltungen:
- EP-A1- 2 181 965
- EP-A1- 2 865 651
- WO-A2-01/89996
- DE-A1- 10 013 964
- DE-A1- 10 111 104
- DE-A1-102009 057 562

## Beschreibung

Die Erfindung betrifft eine Vorrichtung zur Wasseraufbereitung nach dem Prinzip der umgekehrten Osmose. Vorrichtungen dieser Art, Umkehrosmose- oder RO Anlagen, werden insbesondere in Verbindung mit Hämodialysegeräten eingesetzt, um aus Leitungswasser ausreichend reines, keimfreies Wasser zur Bereitung der Dialysierflüssigkeit zu gewinnen.

Die DE 10013964 A1 offenbart eine Anlage zur Versorgung von Dialysegeräten mit Permeat, bei der die Hochdruckpumpe des Umkehrosmosemoduls das Permeat direkt in die Ringleitung pumpt, wo es durch die Umwälzpumpe zu den Entnahmestellen der Dialysegeräte strömt. Dies erfolgt unabhängig davon, ob die Dialysegeräte große oder kleine Mengen Permeat entnehmen, so dass der Energiebedarf unverändert hoch bleibt. Zur periodischen Heißreinigung hat die vorbekannte Anlage einen Heißwasserbehälter, der während des normalen Dialysierbetriebs befüllt wird. Immer dann, wenn relativ geringe Mengen von Permeat durch die Entnahmestellen abgezogen werden, erfolgt die Befüllung des Heißwasserbehälters dadurch, dass ein im Zulauf zu dem Heißwasserbehälter angeordnetes Überströmventil öffnet, bis der Heißwasserbehälter bis zu einem oberen Niveaugeber befüllt ist. Sobald der Heißwasserbehälter mit Permeat gefüllt ist und mittels einer Heizeinrichtung aufgeheizt ist, kann eine Heißreinigung der Anlage stattfinden. Hierzu wird das Umkehrosmosemodul von der Ringleitung abgekoppelt, das Heißwasserventil geöffnet und das heiße Permeat in die Ringleitung gepumpt, wo es zirkuliert.

Die Erfindung zielt insbesondere darauf ab, Ressourceneinsparungen in Form elektrischer Energie und Rohwasser bei dem Betrieb der Umkehrosmose und oder auch der Dialysemaschinen zu erreichen. Weitere Ziele sollen aber auch in minimalen Raumansprüchen, als auch in der Ausfallsicherheit der Reinstwasserversorgung liegen.

Diese Aufgabe wird durch die Merkmale des Hauptanspruches gelöst. Weitere Merkmale und Ausgestaltungen ergeben sich aus den Unteransprüchen und der nachfolgenden Beschreibung von Ausführungsbeispielen in Verbindung mit den Abbildungen. Dabei zeigen:
- Fig. 1: das Schema einer Umkehrosmoseanlage mit den Austattungsmerkmalen entsprechend der Erfindung;
- Fig. 2: Ausgestaltungen wesentlicher Baugruppen gemäß der Erfindung.

Bei dem Schema der Umkehrosmoseanlage entsprechend der Erfindung sind es insbesondere die Anordnung der eingesetzten Hauptbaugruppen, deren konstruktive Ausgestaltung und das Zusammenwirken mit Aktoren und Sensoren, die eine erhebliche Ressourceneinsparung in allen Programmschritten - Versorgen der Dialysegeräte mit Reinstwasser, Reinigen der Ringleitung, Reinigen der Umkehrosmose - erzielen.

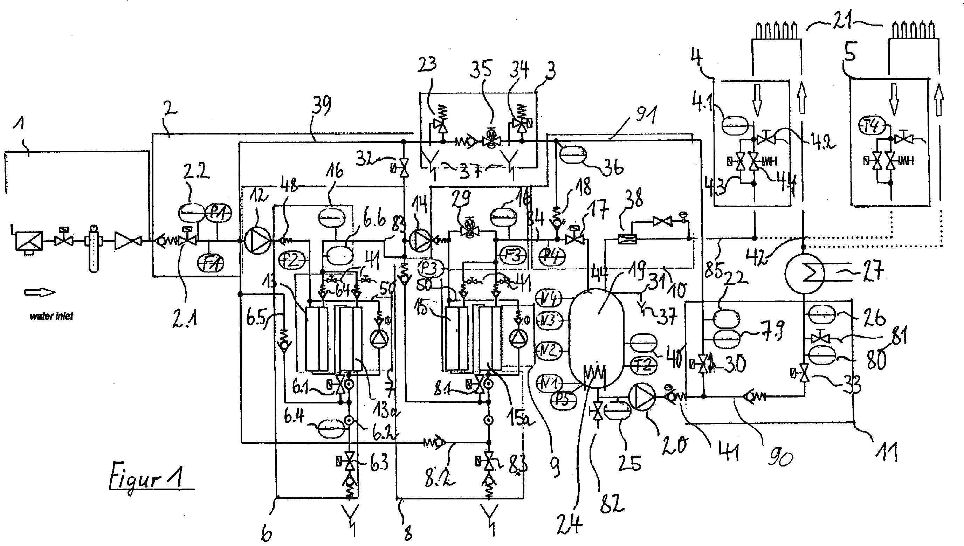

Das in Fig. 1 gezeigte Schema veranschaulicht das Zusammenspiel der wesentlichen Hauptbaugruppen. Anhand des Schemas wird aufgezeigt wie die Ressourceneinsparung in den Programmschritten erzielt wird.

Eine Umkehrosmose nach dem Stand der Technik arbeitet beim Versorgen der Dialysegeräte mit Reinstwasser entweder im Tankbetrieb d.h. ein Reinstwasser Tank wird im Start-Stop Betrieb gefüllt, vom Tank aus versorgt eine Permeatverteilungspumpe die Dialysegeräte, oder die Umkehrosmose fördert das Reinstwasser direkt zu den Dialysegeräten.

Beide Verfahrensvarianten sind hinsichtlich des realen Dialysebetriebes, bei dem die Anzahl der angeschlossen Behandlungsplätze je nach Wochentag und Tageszeit stark variieren kann nicht optimal.

Zum einen ist das Tankvolumen und die Speisekapazität der Umkehrosmose auf einen optimalen Arbeitspunkt abzustimmen, der bei schwankenden Auslastungen entweder zu einem häufigen Start/Stop, oder zu langen Stillstandszeiten der Umkehrosmose, mit dem Risiko einer Verkeimung, führt. Auch Umkehrosmosen für Direkteinspeisung können niedrige Auslastungen trotz Wirkungsgradsteuerung nur ungenügend ausgleichen, weil z.B. die Pumpen auf die maximale Förderleistung auszulegen sind.

Vorteilhafterweise ist der Tank deshalb in seinem Volumen auf maximal 200 I begrenzt. So kann z.B. die Umkehrosmose bei einer möglichen Versorgung von 50 Behandlungsplätzen und einer Umkehrosmosekapazität von ca. 2500l/h ohne Start/Stop Betrieb über den Tank, mit einer zusätzlichen Permeatverteilerpumpe Reinstwasser zu den Dialysegeräten fördern. Leicht schwankende Entnahmemengen sind auszugleichen, indem bei Erreichen eines oberen Tankfüllstandes die Tankzufuhr schließt und überschüssiges Permeat zum Eingang der Umkehrosmose zurückgeführt wird. Bei minimalen Entnahmen z.B. beim Versorgen von nur wenigen Behandlungsplätzen mit Reinstwasser - grundsätzlich jedoch bei ca. 30% der Nennleistung wird die Umkehrosmose in einen Tankbetrieb d.h. Start/Stop Betrieb geschaltet.

Die vorgenannte Leistung i.H. von 30 % der Nennleistung ist eine aus dem realen Dialysebetrieb abgeleitete Größe.

Bei ca. 5000 Jahresbetriebsstunden einer Umkehrosmose mit ca. 2500l/h im Dialysebetrieb sind ca. 22000 Behandlungen möglich. Dabei werden erfahrungsgemäß ca. je 50% in Nennleistung und ca. 50% mit lediglich 30% der Nennleistung erbracht.

Während die Pumpen einer Standard Umkehrosmoseanlage auch im Teillastbetrieb ihre volle Leistung für die Dauer der Dialysebehandlung erbringen und einen Konzentratabfluss, trotz Wirkungsgradregelung auch bei geringer Entnahmemengen aufweisen, werden erfindungsgemäß die Pumpen und der Konzentratabfluss bei vollen Tank gestoppt und die Dialysegeräte mittels einer geringen Leistung beanspruchenden Permeatverteilerpumpe versorgt

Bei einer angenommenen Umkehrosmosekapazität von ca. 2500l/h und Wasserpreisen von ca. 5 €/m³, einem Strompreis von 0,30 €/kWh und ca. 2500 Betriebsstunden p.a. mit auf ca. 30% reduzierten Behandlungsplätzen, ergeben dies Jahreseinsparungen von ca. 5 T€ Strom (2 Pumpen a'4,2kW/h x 2000hx ,3€/kWh) und 5T€ Wasser (0,5m³×2000h×5€/m³). Also insgesamt ca. 10T€ p.a. Die elektrische Heizleistung einer Dialysemaschinen zur Aufbereitung der Dialysierflüssigkeit/ Dialysebehandlung beträgt bei einer Temperaturerhöhung von ca. 15°C auf 37 °C ca. 5,12 kWh.

Bei den vorgenannten Betriebsdaten und einem typischen Wasserverbrauch von ca. 200 I pro Dialysebehandlung und ca. 22000 Behandlungen p.a. summiert sich dies zu ca.100.000 kWh p.a.

Ziel der Erfindung ist es die elektrische Energie die zum Aufbereiten der Dialysierflüssigkeit benötigt wird, zumindest teilweise durch Primärenergie (ÖL, Gas, Sonne, Wind) zu ersetzen.

Im Preisvergleich zum Einsatz elektrischer Energie, ergibt sich mittels Wärmeübertragung durch Primärenergie in den Tank und oder durch zusätzliche Wärmetauscher in der Ringleitung des Permeates, je nach Art der Primärenergie (Öl, Gas; Sonne) einen Einspareffekt gegenüber den Stromkosten von bis 32T€ p.a.

(5,12 kWhl pro Behandlung : 4 Std/Behandlung= 1,28kW× 48 Behandlungsplätze= 61,44kW benötigte Primärenergie)

Praktisch realisiert werden kann dieser Wärmeeintrag, durch Primärenergie, je nach Tankgröße ganz oder teilweise in den Tank (doppelwandig und oder Wärmetauscher) oder zusätzliche weitere Ringwärmetauscher.

Als weitere Möglichkeit kann die Versorgung der später noch zu beschreibenden elektrischen Tankheizer mit Primärenergie in Betracht gezogen werden.

Die dargestellte Umkehrosmose ist doppelstufig. Zum einen um eine möglichst hohe Reinstwasserqualität zu erzielen und zum anderen um beim Ausfall einer Stufe die Versorgungssicherheit der Behandlungsplätze aufrecht zu erhalten. Darüber hinaus kann bei einem auftretenden Defekt an elektrischen, hydraulischen oder mechanischen Komponenten, die erste oder die zweite Stufe jeweils einzeln ohne die elektronische Hauptsteuerung betrieben werden.

Das Starten des Notbetriebs erfolgt durch einen Schlüsselschalter, welcher eine von der Hauptsteuerung unabhängige Relaisschaltung aktiviert an der eine unabhängige Leitfähigkeits- und Temperaturüberwachungseinrichtung und ebenso von der Hauptsteuerung unabhängige Ventilschaltung angeschlossen ist.

Für die Funktion der RO-Membranen ist die Überströmung der Primärseite von erheblicher Bedeutung. Unter Berücksichtigung der Rohwasserqualität sind die Betriebsbedingungen der Membranen so einzustellen, dass der über die Primärseite eingestellte Konzentratfluss ein mehrfaches des zu den Dialysegeräten geförderten Permeatflusses beträgt.

Der für die Membranfiltration notwendige Druck und die Förderung des Permeates wird durch die Umkehrosmose Pumpen realisiert. Vorteilhafterweise können Zirkulationspumpen zwischen Primäreingang und Primärausgang (Konzentratausgang) der Membranen vorgesehen sein, um eine optimale Überströmung u. damit eine lange Lebensdauer der Membranen zu erzielen.

Der noch wichtigere Vorteil liegt dabei auf dem, um ca. 70%, reduzierten Stromverbrauch gegenüber einer Umkehrosmose ohne Zirkulationspumpen . Typisch sind bei vorgenannter Nennleistung 2 Pumpen a'4,2kW/h eingesetzt. Bei 5000 Betriebsstunden und 0,3€/kWh werden ca. 8T€ p.a. eingespart.

Dabei zeigt Figur 1 eine doppelstufige RO, die sowohl in der ersten als auch in der zweiten Stufe zur Leistungsverdopplung mit je zwei Membranen (13 /15) ausgestattet ist. Das zugeführte Rohwasser wird über ein Eingangsventil (2.1) und die LF-Messung (2.2) zur Pumpe (12) geführt und gelangt als Reinstwasser der ersten Stufe (83) zur Pumpe (14), die via Membrane (15) hochreines Permeat über eine Leitung (84), das Tankfreigabeventil (17) und den Tankeinlauf (44) in den Permeattank (19) führt.

Von dort wird das hochreine Permeat, über Pumpe (20), Drucküberwachung (80), Probeentnahme (81), Flussmesser(26) und Leitung (42) zu den Dialysegeräten(21) geführt. Überschüssiges Permeat kann über die Rücklaufeinheiten (4/5), einem Druckhalteventil (4.4) weiteren Probeentnahmeventil (4.2) und Temperatursensor (4.1) zurück in den Tank (19) geleitet werden. Ventil (4.3) öffnet bei Anforderung um die Druckhaltung des Ringes z.B. bei Reinigungsprogrammen aufzuheben. Rücklaufeinheit (5) ist bei Installation einer zweiten Permeatversorgungsleitung identisch aufgebaut.

Bei entsprechend großen Permeatentnahmemengen durch die Dialysegeräte (21) wird Tank (19) lediglich als Durchlaufeinheit genutzt. Bei geringeren Entnahmen ist eine Abschaltung des Ventils (17) in Abhängigkeit der vier Niveauschaltungen am Tank möglich, dabei kann das Permeat über Leitung (39) zurück zur Pumpe (12) fließen. Mit Vorteil werden jedoch die Pumpen (12/14) abgeschaltet- um Energie/Wasser zu sparen- bis die Niveauschaltung des Tanks (19) eine erneute Anforderung registriert.

Das erzeugte Permeat, das über Leitung (42) zur Aufbereitung der Dialyseflüssigkeit für Dialysegeräte (21) genutzt wird, kann mittels Primärenergie über Wärmetauscher (24127) bzw. und oder elektrischen Heizer (40) -auch über Primärenergie- nahezu auf die erforderliche Temperatur zur Aufbereitung der Dialysierflüssigkeit vorgeheizt werden, um elektrische Heizenergie der Dialysegeräte einzusparen. Dazu sind die in Funktionseinheit (11) benannten Aktoren und Sensoren als auch die Rezirkulation über Leitung (42) und Tank (19) mittels Pumpe (20) und die Überwachung (25) erforderlich.

Damit die Pumpen (12/14) ein Minimum an elektrischer Leistung aufbringen müssen, ist deren Kapazität so ausgelegt, dass ein effizienter Betrieb mit geringem Abfluss über Ventile (6.3 und 8.3) und der maximal erforderlichen Permeatmenge möglich ist. Die für die Membrane (13/15) erforderliche konstante, primärseitige Überströmung wird aussschließlich über Zirkulationspumpen (7.9) gewährleistet.

Bei Ausfall der Umkehrosmose, beispielsweise der ersten Stufe (12/13) wird das Rohwasser über Ventil (32) zu Pumpe (14) geführt von dort kann es bei funktionstüchtiger Pumpe (20) in Ringleitung (42) fließen. Bei Ausfall von Pumpe (20) besteht alternativ die Möglichkeit Ventil (17) zu schließen und das Permeat über Überströmventil (18) und Leitfähigkeits-, Temperaturmessung (36) und bidirektionales Ventil (30), das zum Reinigen der Membran in Gegenrichtung durchströmbar ist, in Ringleitung (42) zu führen. Überschüssiges Permeat fließt in Tank (19) zurück und geht via Überlauf (31) zum Abfluss (37). In diesem Fall wird das motorisch angetriebene Ventil (35) geschlossen, bei Ausfall von Stufe 2 (14/15) wird das Permeat über das geöffnete motorisch angetriebene Ventil (29) und dem vorher beschriebenen Verfahren zu den Dialysegeräten geleitet. Ventile (29/35) sind auch manuell zu betätigen.

Die Leitfähigkeits-,Temperaturmessung (36) und Ventile (30/33) können unabhängig von der Hauptsteuerung betrieben werden um einen sicheren Notbetrieb zu gewährleisten. Ventil (30) ist sicher bidirektional zu betreiben um zum einen den Notbetrieb und zum anderen die Reinigung der Membranen mit heißem Wasser durchzuführen.

Zur Reinigung der Permeatversorgungsleitung (42/85) wird das Tankvolumen mittels Heizer (40) elektrisch oder über Primärenergie aufgeheizt. Ebenso besteht alternativ die Möglichkeit einer Erwärmung über Wärmetauscher (24/27). Pumpe (20) zirkuliert das erwärmte Permeat auf die erforderliche Temperatur, die mittels Temperatursensoren (25/ 4.1) registriert wird. Flussmesser (26) dient als Überwachung beispielsweise zum Leckageschutz oder Entnahmen durch die Dialysegeräte (21).

Zur Reinigung der Membranen wird das im Tank (19) befindliche Permeat, wie vorher beschrieben, erwärmt. Ventil (33) geschlossen; das bidirektionale Ventil (30) geöffnet, Pumpe (20) fördert das Permeat wahlweise über Ventil (32) zur 2. Stufe oder zur Pumpe (12) der 1. Stufe und über diese zur 2.Stufe. Dadurch können die Stufen wahlweise unabhängig, oder zusammen heißgereinigt werden. Dazu werden die Hochdruckbypassventile (6.1./8.1) geöffnet, die Pumpen (12 / 14) mit niedrigem Druck membranschonend betrieben, entweder drehzahlgeregelt oder mit großem Vorteil, entgegengesetzt drehend umgeschaltet, um einen niedrigen Konzentratdruck zu erzielen. Nach einem kurzen Spülvorgang der Membranprimärseiten (13/15), werden die Abflussventile (6.3 und 8.3) geschlossen. Pumpen (7 und 9) zirkulieren,

Um eine chemische oder chemothermische Desinfektion der RO-Anlage durchzuführen kann Desinfektionsmittel über Ansaugstecker (69) dosiert und mittels Venturi oder andere Dosierpumpen in Tank (19) eingebracht werden. Von dort wird es über Ringleitung (42) bzw. alternativ bei abgesperrter Ringleitung (42) über das bidirektionale Ventil (30) zur RO gebracht, um dort verteilt zu werden.

Restvolumen im Tank (19) wird durch Zuführen vom frischen Permat verdünnt und über Ventil (34) zum Abfluss gefördert. Ventil (34) hat neben der elektrischen Betätigung auch eine mechanische Überströmung, um beispielsweise bei Notversorgen die Leitung (42) vor Überdruck zu schützen.

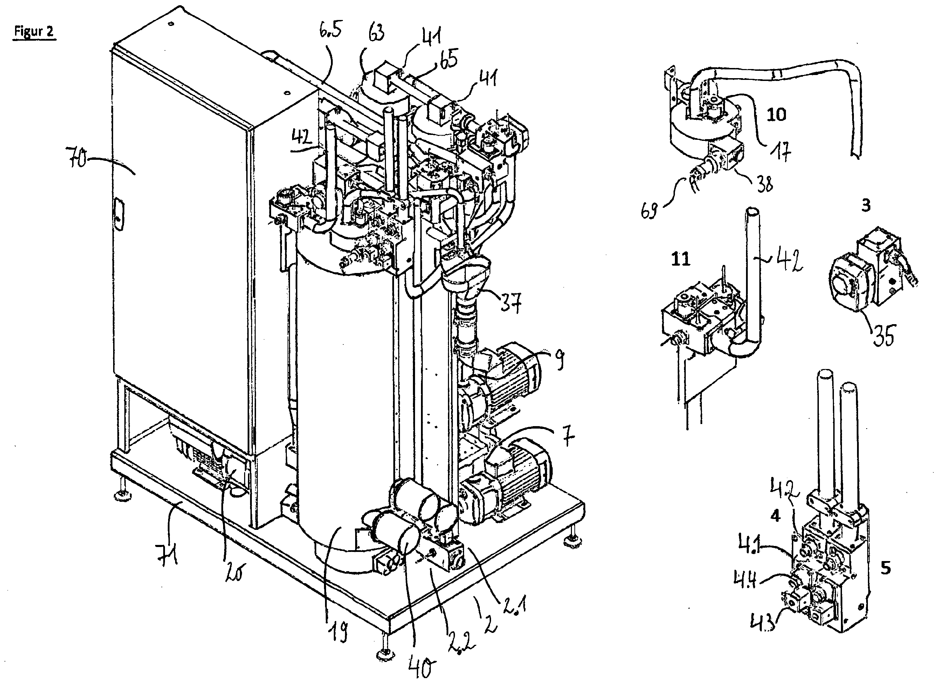

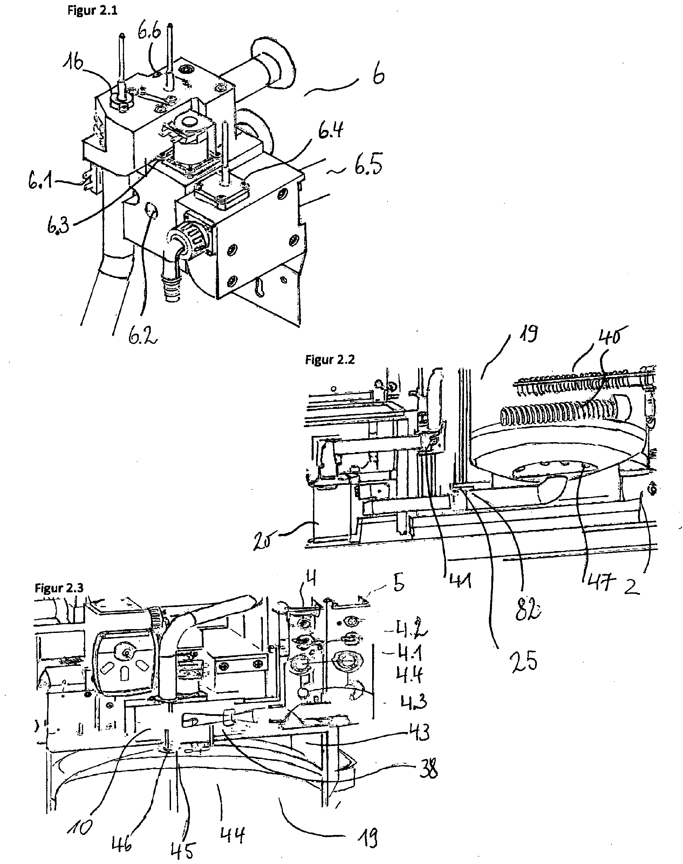

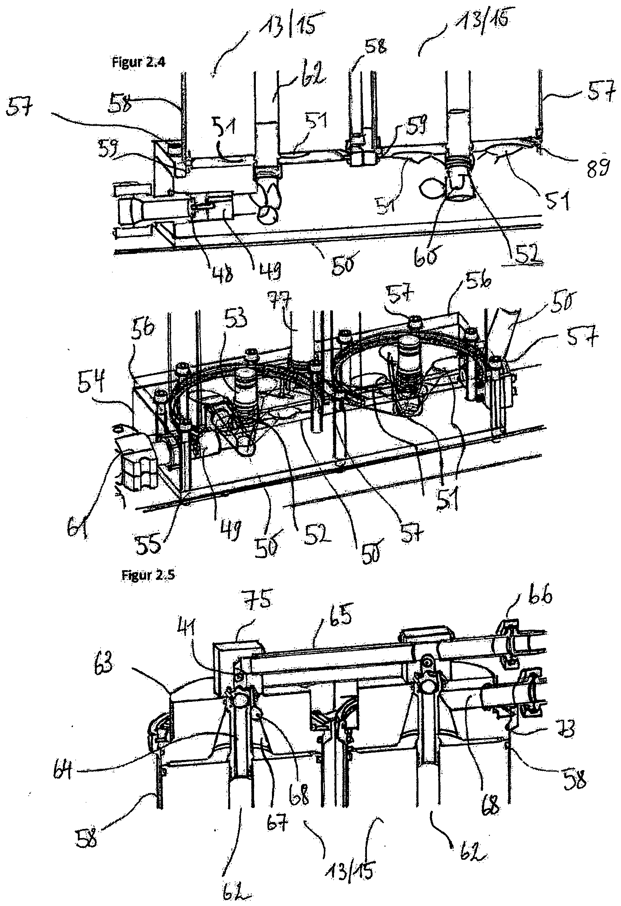

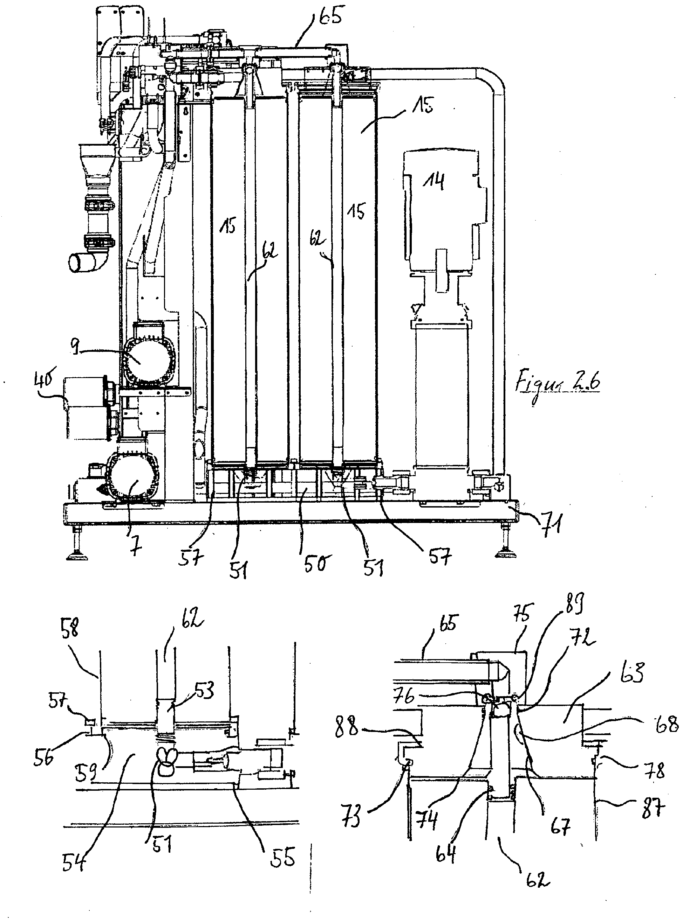

Ebenso schützt Ventil (23) das System vor zu hohen Rohwassereingangsdruck. Figur 2 zeigt eine Perspektive der RO-Anlage um den räumlichen Aufbau bzw. die Kompaktheit darzustellen. Mit einer Länge von ca. 1200mm, ca. Breite 900mm und Höhe ca. 1700mm wurden nahezu Palettenmaße erreicht, damit ist die Anlage türgängig, hat geringe Aufstellmaße und niedrige Frachtkosten.

Die dargestellte doppelstufige Anlage erbringt ca. 2300 - 2500 Liter Reinstwasser pro Stunde.

Beispielhaft sind einige der maximal 15 Baugruppen auch in Figur 2.1 dargestellt, um die Kompaktheit zu verdeutlichen.

Figur 2.2 zeigt einige Details des Tanks 19 mit den seitlich angebrachten Heizern (40) und den über dem Bodenablauf angebrachten Zyklonverhinderer (47), der bei einer stark saugenden Pumpe (20), niedrigen Füllständen im Tank (19) und hohen Temperaturen der Flüssigkeit erforderlich ist. Die Zyklonverhinderung vermeidet einen Lufteintrag in Pumpe (20) und in das System.

Figur 2.3 zeigt einen Schnitt des Tankeinlaufblocks (10). Nicht dargestellt ist, die in der Mitte des Tankeinlaufblocks (10) befindliche Entlüftungsbohrung, die Luft bzw. überlaufende Flüssigkeit über Leitung (31) zur Drainage (37) führt. Mit Vorteil wird sowohl das zufließende- , als auch das rückfließende Permeat, im Tankeinlauf (44) über radialangeordnete Auslasskanäle (45) so in eine ringförmige Auslassnut (46) geführt, dass die Tankinnenwand vollumfänglich benetzt und ohne Totraum gespült werden kann. Ebenso nicht dargestellt ist ein zusätzlicher im Tankdeckel angebrachter Zugang für einen Schwimmerschalter mit mindestens 4 Kontaktmeldungen.

Figur 24 zeigt den Modulblock (54) mit den daran befestigten Modulrohren (58) und den darin befindlichen Membranen (13/15). Das dünnwandige Modulrohr (58) ist dabei mit einer prismatischen Flanschplatte (56) verschweißt und wird mit Schrauben (57) mit der Modulbodenplatte (55) verschraubt. Zur Abdichtung liegt ein Dichtring (89) in einer umlaufenden Nut des Modulblockes (54) und dichtet gegen die innen kreisförmig ausgeschnittenen und bearbeiteten Dichtfläche der Flanschplatte (56). Dazu muss die Materialdicke der Flanschplatte mindestens größer 10 mm sein.

Das Permeatsammelrohr (62) wird mittels Stopfen (53) unten verschlossen und mit Stützfeder (52) schwimmend gehalten, um hydraulische Stöße auszugleichen und Material Ausdehnungen der Membranen (13/15) aufzunehmen. Die Roh-Wasserzuführung über Pumpe (12) kann über Rückschlagventil (48) und Eingangsströmungsverteiler (49) erfolgen und wird über mindestens 4 kreisförmig angeordnete schräge Bohrungen (51) auf die untere Stirnseite der Membran geführt.

Die Leitung (50) der Zirkulationspumpen (7/9) wird ebenfalls im Modulblock (54) geführt.

Dabei wird das zugeführte Rohwasser mit der Flüssigkeit der Zirkulationspumpen (7/9) gemischt und durch die spiralförmig gewickelte Membranen, als auch über einem Bypass-Strom im Ringspalt (87) die Druckrohrinnenseiten und Membran-Außenseiten geführt.

Das über die Membran erzeugte Permeat wird über Permeatsammler (62) zu Membranrückschlagventil (64) im Modulkopf (63) geleitet. Membranrückschlagventil (64) stellt dabei eine dichtende Verbindung her. Rückschlagventil (64) beinhaltet eine Kugel (66) und Dichtung (72), um die Membran vor Rückflüssen zu schützen.

Permeatsammler- Probeentnahmeblock (75) steckt auf Membranrückschlagventil (64) und wird mittels Zylinderstiften (89) darauf gesichert. Durch die Permeatsammler- Probeentnahmeblock (75) befindliche Probeentnahme (41) ist eine selektiver Test für jede Membrane möglich.

Von Permeatsammler- und Probeentnahmeblock (75) wird das Permeat über Permeatverbinder (65) weitergeführt.

Sowohl der Modulblock (54) als auch der Modulkopf (63) besitzen Ausfräsungen (74) damit bei anliegender Membranstirnfläche die Flüssigkeit aus Ringspalt (47) fließen kann. Das austretende Konzentrat wird am Modulkopf (63) über einen Innenkonus (67) und Ausgangsbohrung (68) abgeleitet.

Modulkopf (63) hat eine umlaufende Nut (73) in der ein Dichtring gegen eine bearbeitete Fläche des Flansches (78) abdichtet.

Die Befestigung des Modulkopf (63) kann mittels Verschraubungen (nicht dargestellt) oder Verklemmung an Flansch (78) erfolgen, dazu wird über Absatz (88) ein Flanschring gelegt und mit dem am Modulrohr angeschweißten Flansch verschraubt, oder eine Nut im Flansch (78) nimmt Sicherungsscheiben auf die den Modulkopf positionieren.

| | |
|---|---|
| 1. | Vorfiltrationseinheit |
| 2. | Wassereingangsgruppe |
| 2.1 | Wassereingangsventil |
| 2.2 | Temperatur und Leitfähigkeitsmessung |
| 3. | Sicherheitsgruppe |
| 4. | Ringrücklauf 1 |
| 4.1 | Temperatursensor Ringrücklauf |
| 4.2 | Probeentnahmeventil |
| 4.3 | Bypassventil |
| 4.4 | Druckhalteventil |
| 5. | Ringrücklauf 2 |
| 6. | Funktionseinheit 1. Stufe |
| 6.1 | Hochdruckdrossel mit Bypassventil |
| 6.2 | Abflussdrossel |
| 6.3 | Abflussventil |
| 6.4 | Abfluss-Flussmesser |
| 6.5 | Konzentratzirkulation |
| 7. | Zirkulationspumpe 1. Stufe |
| 8. | Funktionseinheit 2. Stufe |
| 8.1 | Hochdruckdrossel mit Bypassventil |
| 8.2 | Permeatzirkulation |
| 9. | Zirkulationspumpe 2. Stufe |
| 10. | Tankeinlaufgruppe |
| 11. | Tankauslaufgruppe |
| 12. | Hochdruckpumpe 1 |
| 13. | Membranen 1. Stufe |
| 14. | Hochdruckpumpe 2 |
| 15. | Membranen 2. Stufe |
| 16. | Temperatur- Leitfähigkeitssensoren |
| 17. | Tankfreigabeventil |
| 18. | Überströmventil |
| 19. | PermeatTank |
| 20. | Permeatversorgungspumpe |
| 21. | Dialysegeräte |
| 22. | Leitfähigkeitsüberwachung Bypasszweig |
| 23. | Sicherheitsventil Wassereingangsdruck |
| 24. | Wärmetauscher Tank |
| 25. | Temperatur-Leitfähigkeitsmessung Tankauslauf |
| 26. | Flussmesser |
| 27. | Wärmetauscher mit Temperaturmess- u. Regeleinrichtung |
| 28. | Permeatrücklaufleitung |
| 29. | Notbetriebsventil 1 Stufe |
| 30. | Bidirektionalesventil Notbetrieb und Heissreinigen Membranen |
| 31. | Überlauf Tank |
| 32. | Notbetriebsventil 2. Stufe |
| 33. | Freigabe Notbetrieb Ventil |
| 34. | Sicherheitsventil Notbetrieb und Entleerungsventil Tank |
| 35. | Sperrventil Notbetrieb |
| 36. | Leitfähigkeits-Temperaturüberwachung Notbetrieb |
| 37. | Abfluss-Sammler |
| 38. | Zudosierung Desinfektionsmittel |
| 39. | Leitung Notversorgen |
| 40. | Heizer |
| 41. | Permeat Probeentnahme |
| 42. | Ringvorlauf |
| 43. | Venturiansaugung |
| 44. | Tankeinlauf |
| 45. | Auslasskanäle Tankeinlauf |
| 46. | Aulassnut |
| 47. | Zyklonverhinderung |
| 48. | Rückschlagventil |
| 49. | Eingangsströmungsverteiler |
| 50. | Zirkulationsleitung |
| 51. | Strömungsaufteilung |
| 52. | Membranstützfeder |
| 53. | Permeatsammlerstopfen |
| 54. | Modulblock |
| 55. | Modulbodenplatte |
| 56. | Modulrohrflanschplatte |
| 57. | Befestigungsschrauben |
| 58. | Modulrohr |
| 59. | Dichtungsnut Modulrohr |
| 60. | Federzentrierung |
| 61. | Pumpenanschluss |
| 62. | Permeatsammer |
| 63. | Modulkopf |
| 64. | Membran-Rückschlagventil |
| 65. | Permeatverbinder |
| 66. | Clamp-Anschluss |
| 67. | Modulkopf Innenkonus |
| 68. | Membran Konzentratausgang |
| 69. | Desinfektionsmittelansaugstecker mit Kontakt |
| 70. | Elektrik/Elektronik |
| 71. | Palletten Rahmen |
| 72. | Modulkopfdichtung oben |
| 73. | Modulkopfdichtung unten |
| 74. | Modulkopfanschlag |
| 75. | Permeatsammler- und Probeentnahmeblock |
| 76. | Rückschlagkugel |
| 77. | Konzentrateingang |
| 78. | Modulrohrflansch |
| 79. | Flussmesser Heissreinigen Membranen und Bypassleitung Überwachung |
| 80. | Ringdrucküberwachung |
| 81. | Permeatentnahmeblock und Probe Permeatvorlauf |
| 82. | Tankentleerung |
| 83. | Permeat 1. Stufe |
| 84. | Permeat 2. Stufe |
| 85. | Permeatrücklaufleitung |
| 86. | Modulrohrdichtring |
| 87. | Ringspalt |
| 88. | Befestigungsanschlag Modulkopf |
| 89. | Befestigung Permeatsammlerblock |
| 90. | weitere Leitung |
| 91. | Bypassleitung |

## Patentansprüche

1. Umkehrmosmose-Anlage mit wenigstens einer Hochdruckpumpe, die Rohwasser wenigstens einem Modulrohr zuführt, in dem eine Membran mit einem Permeatsammelrohr angeordnet ist, wobei das Modulrohr einen Permeatausgang aufweist,
**dadurch gekennzeichnet,**
**dass** der Permeatausgang des wenigstens einen Modulrohres (58) über eine erste Leitung (84) mit einem Permeattank (19) verbunden ist, wobei in die erste Leitung (84) ein Tankfreigabeventil (17) eingeschaltet ist, das in Abhängigkeit von Niveauschaltungen am Permeattank (19) abschaltbar ist, so dass das Permeat über eine Leitung (39) zurück zu der Pumpe (12) fließt, dass der Permeattank (19) über eine weitere Leitung (90), in die eine Permeatversorgungspumpe (20) eingeschaltet ist, mit einer Ringvorlaufleitung (42) in Verbindung steht, an die mehrere Dialysegeräte anschließbar sind, die zur Aufbereitung von Dialysierflüssigkeit Permeat aus der Ringvorlaufleitung (42) entnehmen, und
**dass** von der ersten Leitung (84) in Strömungsrichtun vor dem Tankfreigabeventil (17) eine Bypassleitung (91) abzweigt, die stromabwärts des Permeattanks (19) und der Permeatversorgungspumpe (20) in die weitere Leitung (90) einmündet.

2. Umkehrosmose-Anlage nach Anspruch 1,
**dadurch gekennzeichnet,**
**dass** die Bypassleitung (91) mit einem Überstromventil (18) und einem bidirektionalen Ventil (30) versehen ist.

3. Umkehrmosmose-Anlage nach Anspruch 1 oder 2,
**dadurch gekennzeichnet,**
**dass** in einer ersten und einer anschließenden zweiten Stufe je eine Hochdruckpumpe (12, 14) in Strömungsrichtung vor vorzugsweise je zwei Modulrohren (58) mit Membranen (13, 15) angeordnet sind,
**dass** der Permeatausgang der ersten Stufe mit der zweiten Hochdruckpumpe (14) verbunden ist,
**dass** der Permeatausgang der zweiten Stufe (15) mit dem Permeattank (19) verbindbar ist, und
**dass** beide Permeatausgänge über Bypassleitungen mit der Ringvorlaufleitung (42) verbindbar sind.

4. Umkehrmosmose-Anlage nach einem Ansprüche 1 bis 3,
**dadurch gekennzeichnet,**
**dass** der Permeattank (19) mit einer Heizeinrichtung (24) versehen ist, mit der das Permeat bis auf 37°C aufheizbar ist.

5. Umkehrosmose-Anlage nach Anspruch 4,
**dadurch gekennzeichnet,**
**dass** der Permeattank (19) doppelwandig ausgebildet ist.

6. Umkehrosmose-Anlage nach einem der Ansprüche 4 oder 5,
**dadurch gekennzeichnet,**
**dass** die Heizeinrichtung (24) ein Wärmetauscher ist, dessen Wärmeträger mit Primärenergie, vorzugsweise mit Gas heizbar ist.

7. Umkehrosmose-Anlage nach einem der Ansprüche 1 bis 6,
**dadurch gekennzeichnet,**
**dass** am Ausgang des Permeattankes (19) ein Zyklonverhinderer (47) angeordnet ist, der die Ausbildung eines Zyklons bzw. eines Lufteintrages in den Ausgang verhindert.

8. Umkehrosmose-Anlage nach einem der Ansprüche 1 bis 7,
**dadurch gekennzeichnet,**
**dass** der Permeattank (19) ein Fassungsvermögen von maximal 200 I, vorzugsweise 100 bis 120 I, hat.

9. Verfahren zur Versorgung von Dialysegeräten mit Reinstwasser mit einer Umkehrosmose-Anlage nach einem der Ansprüche 1 bis 8,
**dadurch gekennzeichnet,**
**dass** bei im Vollastbetrieb entnommenen Permeatmengen der Permeattank (19) bei laufenden Hochdruckpumpen (12, 14) als Durchlaufeinheit genutzt wird.

10. Verfahren zur Versorgung von Dialysegeräten mit Reinstwasser nach Anspruch 9,
**dadurch gekennzeichnet,**
**dass** im Teillastbetrieb von bis zu 30% der Nennleistung der Anlage alle Hochdruckpumpen (12, 14) bei vollem Permeattank (19) gestoppt werden, und
**dass** das Permeat des Permeattankes (19) nur durch die Permeatversorgungspumpe (20) zur Ringvorlaufleitung (42) gepumpt wird.

11. Verfahren zur Versorgung von Dialysegeräten mit Reinstwasser nach einem der Ansprüche 9 bis 10,
**dadurch gekennzeichnet,**
**dass** im Notbetrieb bei Ausfall der Permeatversorgungspumpe (20) das Permeat durch die Bypassleitung zur Ringvorlaufleitung (42) gepumpt wird, wobei die Ventile (17, 30) von einer autarken Steuereinrichtung geschaltet werden.

## Claims

1. A reverse osmosis system with at least one high pressure pump, which supplies raw water to at least one module pipe, in which a membrane with a permeate collecting pipe is arranged, wherein the module pipe has a permeate outlet, **characterised in that** the permeate outlet of the at least one module pipe (58) is connected via a first conduit (84) to a permeate tank (19), wherein connected into the first conduit (84) there is a tank opening valve (17), which may be shut off in dependence on level switches on the permeate tank (19), so that the permeate flows back via a conduit (39) to the pump (12), that the permeate tank (19) is connected via a further conduit (90), connected into which there is a permeate supply pump (20), to a ring feed conduit (42), to which a plurality of dialysis devices may be connected, which remove permeate from the ring feed conduit (42) for the preparation of dialysis liquid, and that branching off from the first conduit (84) before the tank opening valve (17) in the flow direction there is a bypass conduit (91), which communicates with the further conduit (90) downstream of the permeate tank (19) and the permeate supply pump (20).

2. A reverse osmosis system as claimed in Claim 1, **characterised in that** the bypass conduit (91) is provided with an overflow valve (18) and a bidirectional valve (30).

3. A reverse osmosis system as claimed in Claim 1 or 2, **characterised in that** arranged in a first and in a subsequent second stage there is a respective high pressure pump (12, 14) before, in the flow direction, preferably two respective module pipes (58) with membranes (13, 15), that the permeate outlet of the first stage is connected to the second high pressure pump (14), that the permeate outlet of the second stage (15) is connectable to the permeate tank (19) and that both permeate outlets are connectable to the ring feed conduit (42) via bypass conduits.

4. A reverse osmosis system as claimed in one of Claims 1 to 3, **characterised in that** the permeate tank (19) is provided with a heating device (24), with which the permeate may be heated up to 37°C.

5. A reverse osmosis system as claimed in Claim 4, **characterised in that** the permeate tank (19) is of double-walled construction.

6. A reverse osmosis system as claimed in one of Claims 4 or 5, **characterised in that** the heating device (24) is a heat exchanger, the heat transfer medium of which may be heated with primary energy, preferably with gas.

7. A reverse osmosis system as claimed in one of Claims 1 to 6, **characterised in that** arranged at the outlet of the permeate tank (19) there is a cyclone preventer (47), which prevents the formation of a cyclone or the introduction of air into the outlet.

8. A reverse osmosis system as claimed in one of Claims 1 to 7, **characterised in that** the permeate tank (19) has a capacity of at most 200 I, preferably 100 to 120 I.

9. A method of supplying dialysis devices with ultrapure water with a reverse osmosis system as claimed in one of Claims 1 to 8, **characterised in that**, with quantities of permeate removed in full load operation, the permeate tank (19) is used, with the high pressure pumps (12, 14) running, as a continuous flow unit.

10. A method of supplying dialysis devices with ultrapure water as claimed in Claim 9, **characterised in that** in partial load operation of up to 30% of the rated output of the system, all the high pressure pumps (12, 14) are stopped when the permeate tank (19) is full and that the permeate in the permeate tank (19) is pumped to the ring feed conduit only by the permeate supply pump (20).

11. A method of supplying dialysis devices with ultrapure water as claimed in one of Claims 9 to 10, **characterised in that** in emergency operation, in the event of failure of the permeate supply pump (20), the permeate is pumped to the ring feed conduit (42) through the bypass conduit, wherein the valves (17, 30) are switched by an independent control device.

## Revendications

1. Installation d'osmose inverse avec au moins une pompe haute pression, qui amène de l'eau brute à au moins un tube de module, dans lequel est disposée une membrane avec un tube de collecte de perméat, dans laquelle le tube de module présente une sortie de perméat,
**caractérisée en ce**
**que** la sortie de perméat de l'au moins un tube de module (58) est reliée à un réservoir de perméat (19) par l'intermédiaire d'une première conduite (84), dans laquelle est mise en marche, dans la première conduite (84), une soupape de libération de réservoir (17), qui peut être mise hors service en fonction de commutations de niveau sur le réservoir de perméat (19) de sorte que le perméat revient à la pompe (12) par l'intermédiaire d'une conduite (39), que le réservoir de perméat (19) est relié, par l'intermédiaire d'une autre conduite (90), dans laquelle une pompe d'alimentation en perméat (20) est mise en marche, à une conduite d'arrivée annulaire (42), à laquelle plusieurs appareils de dialyse peuvent être raccordés, lesquels prélèvent du perméat provenant de la conduite d'arrivée annulaire (42) pour préparer du liquide de dialyse, et
**qu'**une conduite de dérivation (91), qui débouche dans l'autre conduite (90) en aval du réservoir de perméat (19) et de la pompe d'alimentation en perméat (20), forme un embranchement avec la première conduite (84) dans une direction d'écoulement devant la soupape de libération de réservoir (17).

2. Installation d'osmose inverse selon la revendication 1,
**caractérisée en ce**
**que** la conduite de dérivation (91) est pourvue d'une soupape de trop-plein (18) et d'une soupape bidirectionnelle (30).

3. Installation d'osmose inverse selon la revendication 1 ou 2,
**caractérisée en ce**
**que** respectivement une pompe haute pression (12, 14) est disposée dans la direction d'écoulement devant de préférence respectivement deux tubes de module (58) avec des membranes (13, 15) dans un premier étage et un deuxième étage qui suit,
**que** la sortie de perméat du premier étage est reliée à la deuxième pompe haute pression (14),
**que** la sortie de perméat du deuxième étage (15) peut être reliée au réservoir de perméat (19), et
**que** les deux sorties de perméat peuvent être reliées à la conduite d'arrivée annulaire (42) par l'intermédiaire de conduites de dérivation.

4. Installation d'osmose inverse selon l'une quelconque des revendications 1 à 3,
**caractérisée en ce**
**que** le réservoir de perméat (19) est pourvu d'un dispositif de chauffage (24), avec lequel le perméat peut être chauffé jusqu'à 37°C.

5. Installation d'osmose inverse selon la revendication 4,
**caractérisée en ce**
**que** le réservoir de perméat (19) est réalisé avec deux parois.

6. Installation d'osmose inverse selon l'une quelconque des revendications 4 ou 5,
**caractérisée en ce**
**que** le dispositif de chauffage (24) est un échangeur de chaleur, dont l'agent caloporteur peut être chauffé avec de l'énergie primaire, de préférence avec du gaz.

7. Installation d'osmose inverse selon l'une quelconque des revendications 1 à 6,
**caractérisée en ce**
**qu'**est disposé sur la sortie du réservoir de perméat (19) un système inhibiteur de cyclone (47), qui empêche la formation d'un cyclone ou d'un apport d'air dans la sortie.

8. Installation d'osmose inverse selon l'une quelconque des revendications 1 à 7,
**caractérisée en ce**
**que** le réservoir de perméat (19) a une contenance de 200 l au maximum, de préférence de 100 à 120 l.

9. Procédé d'alimentation d'appareils de dialyse en eau pure avec une installation d'osmose inverse selon l'une quelconque des revendications 1 à 8,
**caractérisé en ce**
**que** le réservoir de perméat (19) est utilisé en tant qu'unité de passage pour des pompes haute pression (12, 14) en fonctionnement en présence de quantités de perméat prélevées à plein régime.

10. Procédé d'alimentation d'appareils de dialyse en eau pure selon la revendication 9,
**caractérisé en ce**
**qu'**en régime partiel jusqu'à 30 % de la puissance nominale de l'installation, toutes les pompes haute pression (12, 14) sont arrêtées lorsque le réservoir de perméat (19) est plein,
et
**que** le perméat du réservoir de perméat (19) est pompé seulement par la pompe d'alimentation en perméat (20) vers la conduite d'arrivée annulaire (42).

11. Procédé d'alimentation d'appareils de dialyse en eau pure selon l'une quelconque des revendications 9 à 10,
**caractérisé en ce**
**qu'**en mode d'urgence, en cas de panne de la pompe d'alimentation de perméat (20), le perméat est pompé par la conduite de dérivation vers la conduite d'arrivée annulaire (42), dans lequel les soupapes (17, 30) sont commutées par un dispositif de commande autonome.
